# EUROPEAN PATENT APPLICATION

(11) **EP 3 195 798 A1**
(43) Date of publication of application: **26.07.2017**
(21) Application number: 17150139.8
(22) Date of filing: 03.01.2017
(51) Int. Cl.: A61B 5/00, A61B 5/01, A61B 5/05

(54) **TOOL, BIOLOGICAL SENSOR, AND SENSING DEVICE**

(30) Priority: 22.01.2016 JP 2016011076
(71) Applicant: FUJITSU LIMITED, Kawasaki-shi Kanagawa 211-8588 (JP)
(72) Inventor: Ishii, Seiki, Kanagawa, 211-8588 (JP); Kim, Najeong, Kanagawa, 211-8588 (JP); Senzaki, Yudai, Tokyo, 144-0051 (JP); Fujita, Hiroyuki, Kanagawa, 211-8588 (JP); Akano, Hiroki, Kanagawa, 211-8588 (JP); Kunitake, Toshihiro, Kanagawa, 211-8588 (JP); Yamaji, Takayuki, Kanagawa, 211-8588 (JP)
(74) Representative: Hoffmann Eitle

(57) **Abstract**

A disclosed tool is configured to be attached to a human body to enable carrying a biological sensor for measurement related to the human body. The tool includes a body part that includes a pocket part capable of accommodating the biological sensor, the body part being configured to be opposed to a chest region of the human body in an attached state of the tool; a shoulder part coupled to the body part, the shoulder part being configured to rest on a shoulder region of the human body in the attached state of the tool; and a torso part coupled to the body part, the torso part being configured to extend around a torso region of the human body in the attached state of the tool.

## Description

### FIELD

The disclosure is related to a tool, a biological sensor, and a sensing device.

### BACKGROUND

An item of underwear, etc., is known from Japanese Laid-open Patent Publication No. 09-201338, for example, in which a flexible biological information transmission device is attached to an inner side of the underwear, and the flexible biological information transmission device includes a loop antenna, a battery, a temperature sensitive sensor, etc., installed on a one-sided flexible substrate.

According to a conventional technique, it is difficult to implement stable measurement with a biological sensor if a user who wears the underwear, etc., which carries the biological sensor, is in motion during the measurement. This is because a position of the biological sensor with respect to a body of the user changes when the user moves such that acceleration in an up-and-down direction or a left-and-right direction acts on the biological sensor or when the user takes a posture such that gravity acts on the biological sensor in a direction away from the body of the user.

Accordingly, It is an object of one aspect of the invention to enable stable measurement even if a user is in motion during the measurement with a biological sensor.

### SUMMARY

According to one aspect, a tool, which is configured to be attached to a human body to enable carrying a biological sensor for measurement related to the human body, is provided, and the tool includes:
a body part that includes a pocket part capable of accommodating the biological sensor, the body part being configured to be opposed to a chest region of the human body in an attached state of the tool;
a shoulder part coupled to the body part, the shoulder part being configured to rest on a shoulder region of the human body in the attached state of the tool; and
a torso part coupled to the body part, the torso part being configured to extend around a torso region of the human body in the attached state of the tool.

### BRIEF DESCRIPTION OF DRAWINGS

Fig. 1 is a perspective view of a tool according to a first embodiment viewed from a front side.
Fig. 2 is a perspective view of the tool from a rear side.
Fig. 3 is a front view of the tool.
Fig. 4 is a rear view of the tool.
Fig. 5 is a diagram explaining a shoulder belt of the tool.
Fig. 6A is a diagram schematically illustrating an opened state of a pocket part.
Fig. 6B is a cross-sectional view along a line B-B in Fig. 6A.
Fig. 6C is a diagram schematically illustrating another example of an opened state of a pocket part.
Fig. 7 is a schematic front view of a user with the tool in an attached state.
Fig. 8 is a front view illustrating a positional relationship, etc., between the shoulder belt and the pocket part.
Fig. 9 is a schematic cross-sectional view along line A-A in Fig. 8.
Fig. 10 is a 5-sided view illustrating an example of a biological sensor.
Fig. 11 is a schematic side view of a user who creeps forward.
Fig. 12 is a perspective view of a tool according to a second embodiment viewed from a front side.

### DESCRIPTION OF EMBODIMENTS

In the following, embodiments are described in detail with reference to appended drawings.

### [First Embodiment]

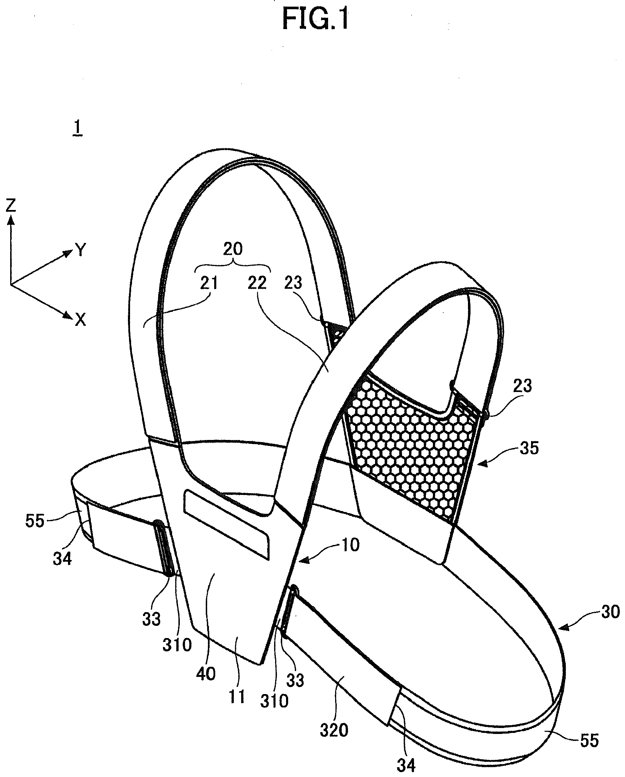

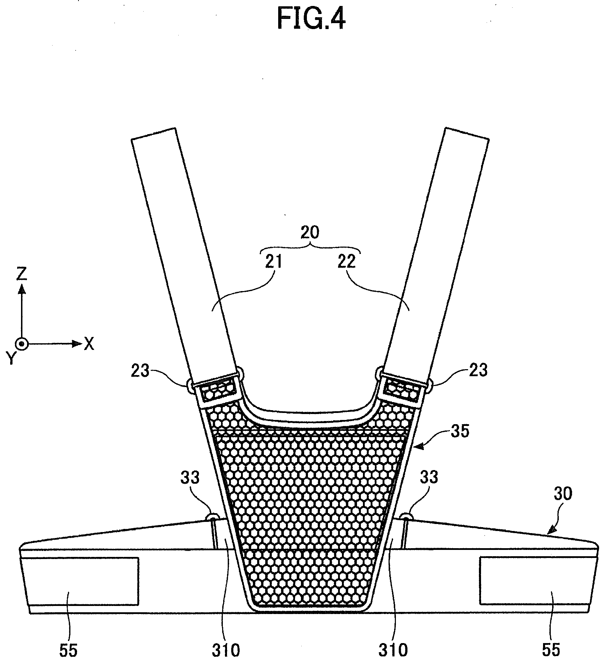

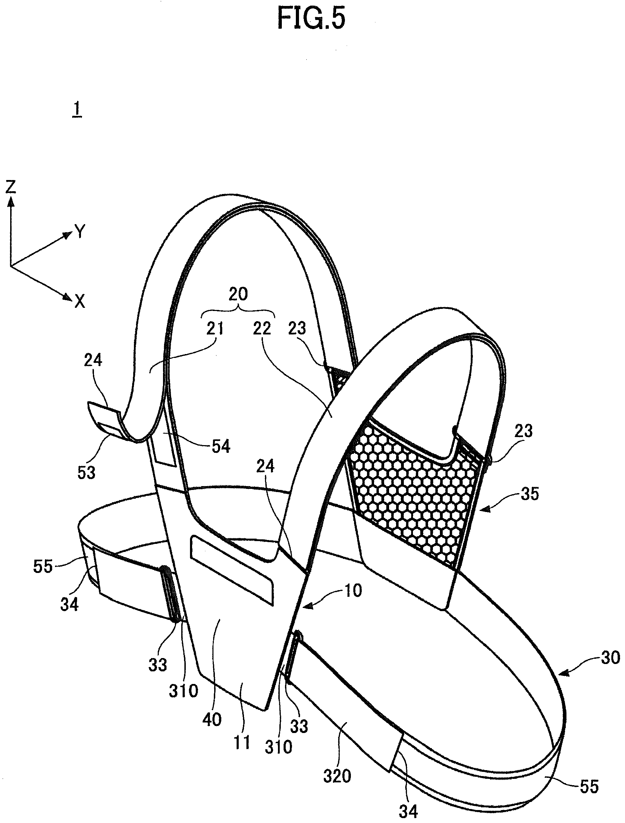

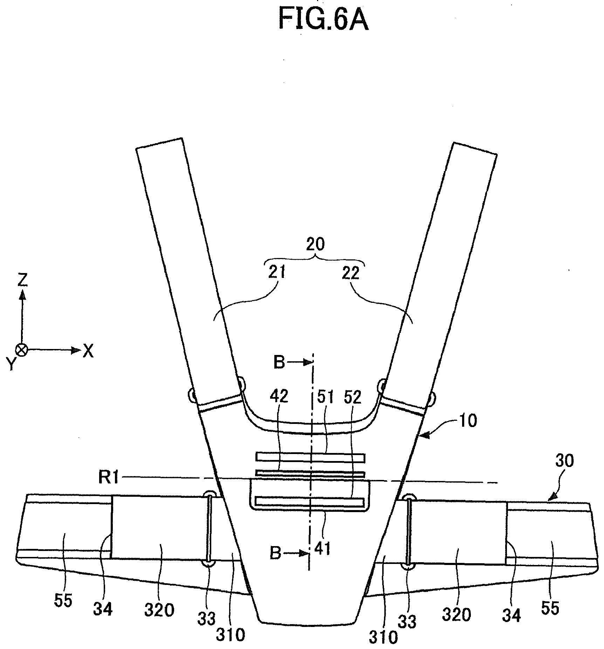

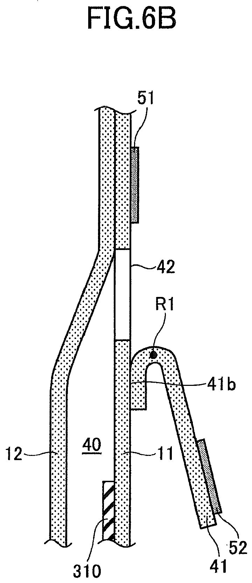

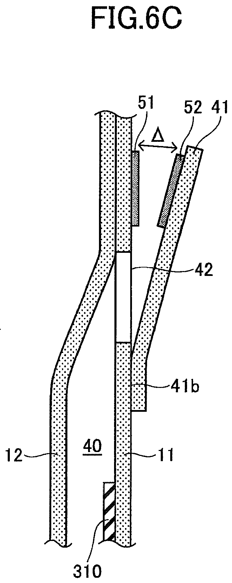

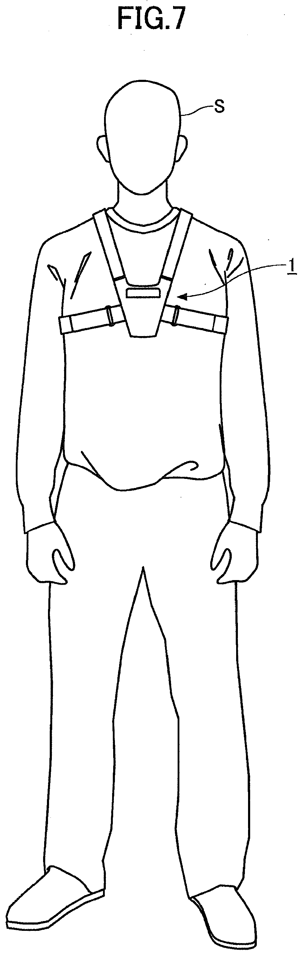

Figs. 1 through 4 illustrate a tool 1 according to a first embodiment, and are a perspective view from a front side, a perspective view from a rear side, a front view, and a rear view, respectively. Fig. 5 is a diagram explaining a shoulder belt 20 of the tool 1. Figs. 1 through 5 illustrate a state in which an opening of a pocket part 40 is closed. Figs. 6A through 6C are diagrams explaining the pocket part 40. Fig. 6A is a schematic front view of the pocket part 40 in a state in which the opening is opened. Fig. 6B is a cross-sectional view along a line B-B in Fig. 6A, and schematically illustrates an example of a state in which the opening of the pocket part 40 is opened. Fig. 6C is a diagram schematically illustrating another example of an opened state of the pocket part 40. Fig. 7 is a schematic front view of a user S with the tool 1 in an attached state.

It is noted that, in the following explanation, directions (left, right, upper, lower, etc.) are defined when the user S with the tool 1 in the attached state is viewed from a front side. In Fig. 1, X direction corresponds to a left-and-right direction, Z direction corresponds to up-and-down direction, and a positive side of Z direction corresponds to an upper side. Further, in following explanation, with respect to a front side and a back side related to the tool 1, the "back side" corresponds to a side closer to the body of the user S and the "front side" corresponds to a side farther from the body of the user S.

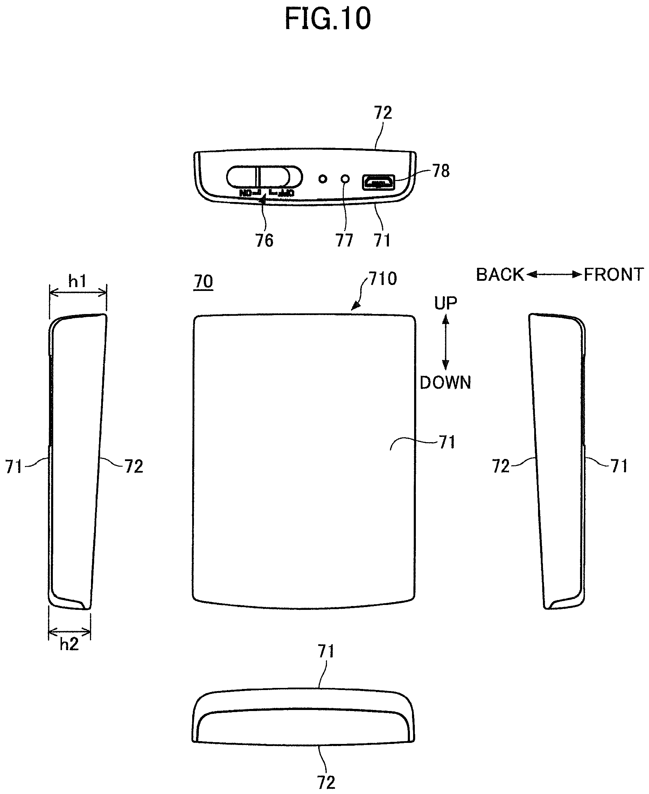

The tool 1 is configured to be attached to the body of the user S to enable carrying a biological sensor 70 (see Fig. 10). The tool 1 may be handled as an item of wear. In the following, "the body of the user S" is also simply expressed by "the body" or "the user S". The tool 1 may be directly attached to the naked body of the user S, or the tool 1 may be attached to the user S via underwear or the like, in terms of comfort. The tool 1 is suitable for being attached to the user S who moves relatively strenuously. This is because, as described hereinafter, even if the tool 1 is attached to the user S who moves relatively strenuously, the tool 1 can reduce the change in a position of the biological sensor 70 with respect to the user S during the strenuous movement. The user S may be a person who exercises (an athlete, for example), a squad member (SDF: Self-Defense Forces personnel in the case of Japan) who performs fire-defense or rescue activities, military personnel, an operator who performs chemical activities such as decontamination or the like, etc. In the following, as an example, the user S is an SDF member, and has the tool 1 attached thereto during a training session.

The tool 1 includes the pocket part 40 (described hereinafter) that can hold the biological sensor 70 (see Fig. 10). The biological sensor 70 performs measurement related to the body of the user S to detect biological information of the user S. Thus, if the user S has the tool 1 attached thereto during the training session, for example, it becomes possible to use the biological sensor 70 to obtain the biological information of the user S during the training session. The biological information to be obtained is arbitrary. According to the first embodiment, as an example, the biological sensor 70 emits radio waves (micro waves, for example) to obtain the biological information (indicative of a respiration, a heart beat, a perspiration amount, etc., of the user S) based on reflected waves. However, the biological sensor 70 may be one of or a combination (in form of a sensor unit) of other sensors such as a sensor that measures heat (a body temperature) of the user S.

The tool 1 includes a body part 10, a shoulder belt 20 (an example of a shoulder part), and a torso belt 30 (an example of a torso part). It is noted that the "belt" represents a portion (member) in a form of a strip (belt) having a longitudinal direction thereof, and the width (a length in a direction perpendicular to the longitudinal direction) thereof is not necessarily constant over the length thereof. The tool 1 may be symmetric in the left-and-right direction, as illustrated in Fig. 1, etc.

The body part 10 may be formed of any materials. The body part 10 is opposed to a chest region of the user S in the attached state. The body part 10 is opposed to the chest region of the user S such that the body part 10 can be opposed to the heart of the user S, for example. According to the first embodiment, as an example, the body part 10 is opposed to a center (in the left-and-right direction) of the chest region of the user S, as illustrated in Fig. 1, Fig. 7, etc. The body part 10 of the user S may have any shape or any size as long as the pocket part 40 can be formed therein and the shoulder belt 20 and the torso belt 30 can be coupled thereto. The shape of the body part 10 may be substantially the same as that of the pocket part 40, for example. According to the first embodiment, as an example, the body part 10 is symmetric in the left-and-right direction, and is in a form of a trapezoid (non-symmetric in the up-and-down direction) whose upper base is longer than a lower base. When the body part 10 is non-symmetric in the up-and-down direction, the user S can immediately recognize the up-and-down direction of the tool 1 at the time of attaching the tool 1 thereto, which eases the attachment of the tool 1. Such an easy attachment is useful, in particular, if the user S has to attach the tool 1 thereto in a short time.

The body part 10 includes the pocket part 40 that is capable of accommodating the biological sensor 70 (see Fig. 10). It is preferred that the pocket part 40 has substantially the same shape as the biological sensor 70. With this arrangement, it becomes possible to reduce the movement of the biological sensor 70 within the pocket part 40, and thus reduce the change in the position of the biological sensor 70 with respect to the user S.

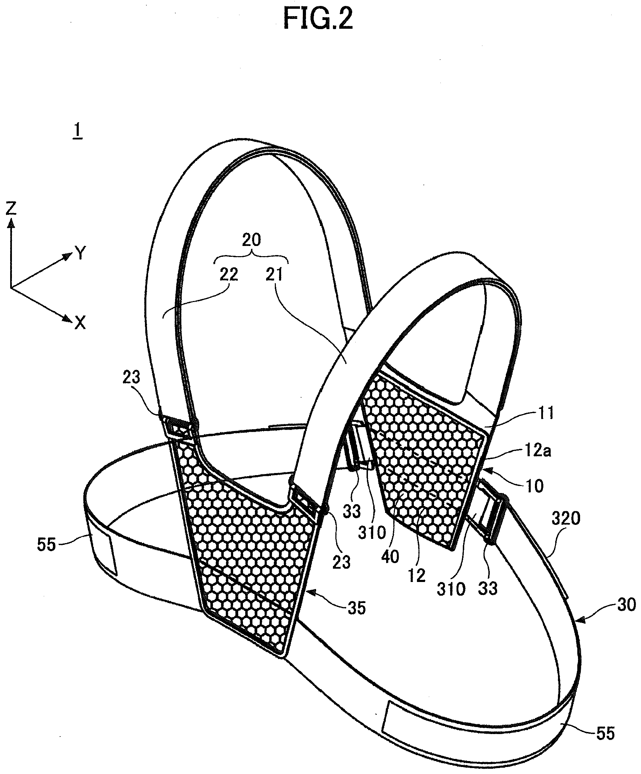

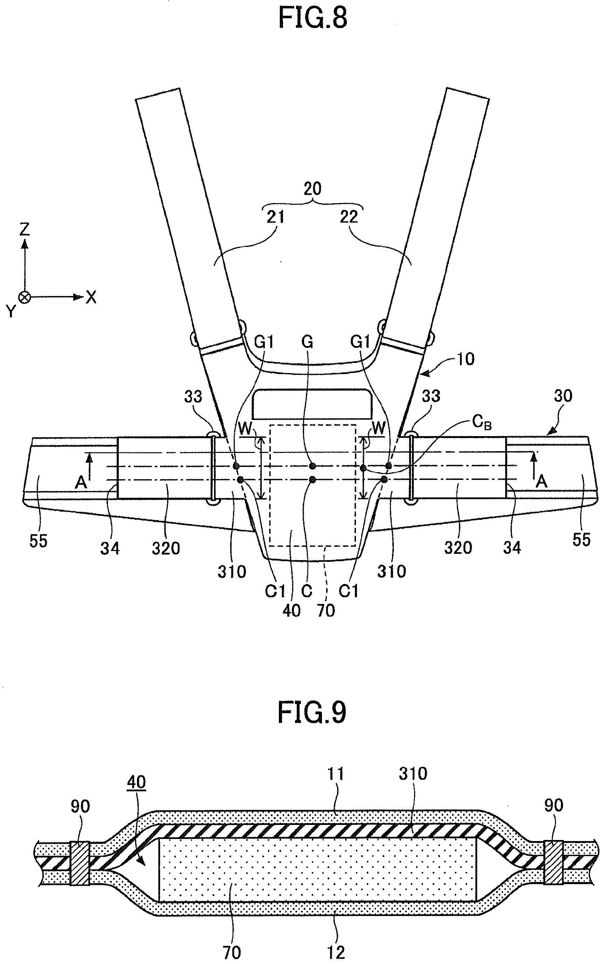

The pocket part 40 may be provided in the body part 10 in any way. For example, the pocket part 40 may be formed by sewing a sack body, which is formed separately from the body part 10, to the front side or the back side of the body part 10, or may be formed together with the body part 10. According to the first embodiment, as an example, the pocket part 40 is formed together with the body part 10. Specifically, the body part 10 includes a front portion 11 and a back portion 12, and the back portion 12 has a peripheral 12a (see Fig. 2) sewed to the back side of the front portion 11 over the length of the peripheral 12a (see a fastened portion 90 in Fig. 9). With this arrangement, the pocket part 40 is formed between the front portion 11 and the back portion 12 and in an area surrounded with the peripheral 12a of the back portion 12.

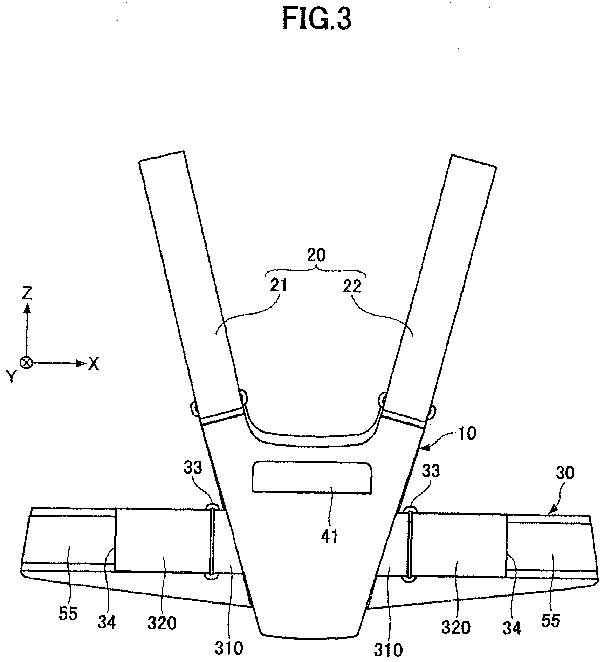

The pocket part 40 may have an opening either on the front side or the rear side of the body part 10; however, it is preferred that the pocket part 40 has the opening on the front side of the body part 10. If the pocket part 40 has the opening on the front side of the body part 10, it becomes possible for the user S to insert or remove the biological sensor 70 with respect to the pocket part 40 in the attached state of the tool 1 without loosening the adjusted shoulder belt 20 or torso belt 30. According to the first embodiment, as an example, the pocket part 40 includes a slit 42 (an example of an opening) formed in the front portion 11, as illustrated in Fig. 3 and Fig. 6A. The slit 42 is formed in such a size that the biological sensor 70 can pass through the slit 42. The slit 42 may have an opening width greater than 0 in the up-and-down direction, or may be in a form of a simple cut in a line. Further, the slit 42 may be reinforced around the opening thereof. The slit 42 is formed on the upper side with respect to the center of the body part 10 in the up-and-down direction, and the area of the pocket part 40 corresponds to the lower side with respect to the slit 42.

It is preferred that the pocket part 40 has a lid 41 that is configured to cover the opening (i.e., implement the closed state). With this arrangement, it becomes possible to reduce a probability that the biological sensor 70 is dropped from the pocket part 40 even when the user S who has the tool 1 attached thereto leans forward, for example. According to the first embodiment, as an example, the lid 41 includes an opening/closing axis R1 that extends in a lateral direction and below the slit 42, as illustrated in Fig. 6A and Fig. 6B. The opening/closing axis R1 is not necessarily a concrete axis, and may be a lower edge of the lid 41 that corresponds to a base portion when the lid 41 is bent. Specifically, the opening/closing axis R1 may be an upper edge of a portion 41b in the lower end of the lid 41, and the portion 41b is sewed to the front portion 11. It is noted that Fig. 6B schematically illustrates the position of the opening/closing axis R1 with a black dot. When the lid 41 is pivoted (i.e., bent or folded) to the lower direction around the opening/closing axis R1 from the closed state of the pocket part 40 illustrated in Fig. 3, the opened state of the pocket part 40 illustrated in Fig. 6A and Fig. 6B is implemented. With this arrangement, it becomes possible to ease the insertion or removal of the biological sensor 70 with respect to the pocket part 40. This is because, in the attached state of the tool 1 and in a state in which the lid 41 is opened, the lid 41 is not in a way of the insertion or removal of the biological sensor 70 even if the user does not use another hand to support the lid 41, which enables the easy insertion or removal of the biological sensor 70 with only one hand. Further, in the state in which the lid 41 is opened, the opening of the pocket part 40 is visible for the user S, which eases the insertion or removal of the biological sensor 70.

It is noted that, in the example illustrated in Fig. 6A, in the opened state of the pocket part 40, the lid 41 is completely folded (rotated by 180 degrees) around the opening/closing axis R1; however, the opening angle of the lid 41 in its opened state may be relatively small, depending on the rigidity of the lid 41, etc. For example, as illustrated in Fig. 6C, in the opened state, the lid 41 may not be folded downward, and thus may extend in an inclined upward direction from the opening/closing axis R1. Even in this case, a clearance Δ between the back side of the lid 41 and the front portion 11 can be used to implement the insertion or removal of the biological sensor 70.

It is noted that, in the closed state of the pocket part 40 illustrated in Fig. 3, the lid 41 covers the slit 42. In this case, a holding mechanism is provided to hold the closed state of the pocket part 40 against gravity acting on the lid 41. In the example illustrated in Fig. 6A, the holding mechanism includes a hook-and-loop fastener 51 on the front portion 11 and a hook-and-loop fastener 52 on the back side (i.e., the back side in the closed state) of the lid 41. The hook-and-loop fasteners 51 and 52 may be Magic Tape (registered trademark) or Velcro (registered trademark). This holds true for other hook-and-loop fasteners described hereinafter. In this case, in the closed state of the pocket part 40 illustrated in Fig. 3, the hook-and-loop fasteners 51 and 52 are coupled to each other. It is noted that the hook-and-loop fasteners 51 and 52 to be coupled to each other are configured such that one of the hook-and-loop fasteners 51 and 52 is of a hook type and the other is of a loop type; however, either one may be of a hook type. This also holds true for other couples of the hook-and-loop fasteners described hereinafter.

It is preferred that the body part 10 is configured such that the front side and the back side can be easily recognized. For example, the body part 10 has different colors between the front side and the back side. The body part 10 may be formed such that the front side and the back side are different materials, instead of or in addition to the different colors between the front side and the back side. With this arrangement, the user S can immediately recognize the front side and the back side of the tool 1 at the time of attaching the tool 1 thereto, which eases the attachment of the tool 1. According to the first embodiment, as an example, as described above, the body part 10 includes the front portion 11 and the back portion 12, and the back portion 12 is formed from a mesh material (see Fig. 2) while the front portion 11 is formed from a nylon material finer than the back portion 12. In this case, in addition to the easy attachment of the tool 1 as described above, the following effects can be obtained, for example. Because the back portion 12, which is located between the user S and the biological sensor 70 in the attached state of the tool 1, is formed from the mesh material, it becomes possible to reduce a probability that the back portion 12 itself interferes with the measurement (a radio wave detection or a heat detection, for example) with the biological sensor 70. Further, because the front portion 11 on the front side is formed from the material of the small fineness, it becomes possible to reduce a probability that undesired objects (sand, etc.) enter the pocket part 40 via the front portion 11 and the front portion 11 is hooked by something during the training session (while the user creeps forward, for example). It is preferred that the mesh material of the back portion 12 is antibacterial and/or deodorant, and/or a material with a quick drying sweat capability (synthetic fibers utilizing a capillary phenomenon, for example), and/or a material with an increased breathability.

The shoulder belt 20 is rested (slung) on a shoulder region of the user S in the attached state of the tool 1, as illustrated in Fig. 7. The shoulder belt 20 may be formed from any material. The shoulder belt 20 is formed from a nylon material, for example. According to the first embodiment, as an example, the shoulder belt 20 has a constant width over the length thereof. The shoulder belt 20 has an end coupled to the body part 10 and the other end coupled to the torso belt 30. According to the first embodiment, the shoulder belt 20 includes shoulder belt positions 21 and 22 which form a pair symmetric in the left-and-right direction. With this arrangement, it becomes possible to increase a constraint capability of the body part 10 with respect to the user S with the shoulder belt 20, in comparison with a case where the shoulder belt 20 includes only one shoulder belt portion to be diagonally slung, for example. The shoulder belt positions 21 and 22 may be diagonally slung (such that the shoulder belt positions 21 and 22 intersect); however, according to the first embodiment, as an example, the shoulder belt positions 21 and 22 are slung in a non-intersecting form.

The shoulder belt 20 has an adjustable length so that the difference in a size of the users S can be accommodated. A length adjustment mechanism is arbitrary. According to the first embodiment, as an example, the shoulder belt 20 can be folded back at flat rings 23. In this case, by adjusting the length of the folded back portion, it becomes possible to adjust the length of a portion from a coupled position on the front side to a coupled position on the rear side. The folded-back side and the non-folded-back side of the shoulder belt 20 are coupled to each other with hook-and-loop fasteners 53 and 54 (see Fig. 5). It is noted that ends 24 (see Fig. 5) on the folded-back side of the shoulder belt 20 may have a thickness and/or a width such that the ends 24 do not escape from the flat rings 23. In this case, at the time of the attachment of the tool 1, even if the shoulder belt 20 is tensioned in a direction such that the shoulder belt 20 is loosen, the shoulder belt 20 is prevented from escaping from the flat rings 23, which leads to the increased convenience at the attachment. Further, in this case, the user S can adjust the shoulder belt 20 in a tension increasing direction by pulling the ends 24 in a downward direction with the hands, which eases the adjustment work.

A coupling between the shoulder belt 20 and the body part 10, and a coupling between the shoulder belt 20 and the torso belt 30 are arbitrary. For example, one of the couplings may be implemented with the sewing, and the other may be implemented via the flat rings 23. According to the first embodiment, as an example, the coupling between the shoulder belt 20 and the body part 10 is implemented by the sewing, and the coupling between the shoulder belt 20 and the torso belt 30 is implemented via the flat rings 23 described above. However, the shoulder belt 20 may be formed together with the body part 10. Further, in the first embodiment, as an example, the shoulder belt 20 is coupled to a back support part 35, which is integrated with the torso belt 30, via the flat rings 23. The back support part 35 has opposite sides of a top portion in the left-and-right direction coupled to the flat rings 23, respectively. It is noted that the back support part 35 and the torso belt 30 may be integrated such that the back support part 35 and the torso belt 30 can be separated or the back support part 35 and the torso belt 30 cannot be separated.

The back support part 35 is opposed to a part of the back of the user S in the attached state. According to the first embodiment, as an example, the back support part 35 is formed from a mesh material, and has substantially the same outline as the body part 10. Specifically, the back support part 35 is in a form of a trapezoid (non-symmetric in the up-and-down direction) whose upper base is longer than a lower base. With this arrangement, the user S can immediately recognize the up-and-down direction of the tool 1 at the time of attaching the tool 1 thereto, which eases the attachment of the tool 1. Further, because the back support part 35 is formed from the mesh material, the breathability on the back side of the user S can be increased. Similarly, it is preferred that the mesh material of the back support part 35 is antibacterial and/or deodorant, and/or a material with a quick drying sweat capability.

It is noted that, if the back support part 35 and the back portion 12 of the body part 10 are formed from the mesh materials, the mesh materials may be the same or different between the back support part 35 and the back portion 12 of the body part 10. For example, the mesh material for the back support part 35 and the mesh material for the back portion 12 may have different colors, different mesh sizes, etc. In this case, the user S can immediately recognize the front side and the back side of the tool 1, which eases the attachment of the tool 1.

The torso belt 30 extends around the torso of the user S in the attached state of the tool 1, as illustrated in Fig. 7. The torso belt 30 may extend around the torso of the user S at the height of the abdomen of the user S in the attached state of the tool 1; however, it is preferred that the torso belt 30 extends around the torso of the user S at the height of the chest of the user S in the attached state of the tool 1. If the torso belt 30 extends around the torso of the user S at the height of the chest of the user S, it becomes unnecessary for the body part 10 to extend downwardly to the height of the abdomen to be coupled to the torso belt 30, which enables the downsizing of the body part 10. According to the first embodiment, the torso belt 30 extends around the torso (around the chest) of the user S at the height slightly lower than the armpit of the user S in the attached state of the tool 1, as illustrated in Fig. 7.

The torso belt 30 may be formed from arbitrary materials; however, it is preferred that at least a part of the torso belt 30 is formed from an extendable material (a rubber material, for example). If at least a part of the torso belt 30 is formed from the extendable material, it becomes possible to reduce the feeling of chest tightness for the user S at the time of breathing, etc., while increasing the contact feeling between the torso belt 30 and the torso of the user S. With this arrangement, even when the user performs such actions as creeping forward, push-ups, for example, it becomes possible to reduce the displacement amount of the biological sensor 70 in the pocket part 40 in a direction away from the user S due to gravity. In other words, it becomes possible to increase a sensor position stabilizing capability described hereinafter. It is noted that, in the first embodiment, as an example, the torso belt 30 as a whole is formed from the extendable material.

According to the first embodiment, as an example, the torso belt 30 has a constant width over the length thereof. It is preferred that the width of the torso belt 30 is different from that of the shoulder belt 20. With this arrangement, the user S can immediately recognize the difference between the torso belt 30 and the shoulder belt 20 at the time of attaching the tool 10 thereto, which eases the attachment of the tool 1. Further, it is preferred that the torso belt 30 has relatively great width, and wider than the shoulder belt 20, for example. Specifically, it is preferred that the width of the torso belt 30 is set such that the pocket part 40 of the body part 10 comes into close and stable contact with the user S in a state where the torso belt 30 is tightened. With this arrangement, the sensor position stabilizing capability described hereinafter can be increased.

Further, it is preferred that the color of the torso belt 30 is different from that of the shoulder belt 20. With this arrangement, the user S can immediately recognize the difference between the torso belt 30 and the shoulder belt 20 at the time of attaching the tool 10 thereto, which eases the attachment of the tool 1. For example, the shoulder belt 20 is khaki, and the torso belt 30 is black.

The torso belt 30 has an adjustable length as is the case with the shoulder belt 20. In the first embodiment, as an example, the torso belt 30 includes a first torso belt 310 coupled to the body part 10, and second torso belt 320. The first torso belt 310 is coupled to the ends of the body part 10 in the left-and-right direction by sewing, for example. The second torso belt 320 has one end coupled to the left side end of the first torso belt 310 and the other end coupled to the right side end of the first torso belt 310 via left and right adjustment mechanisms, respectively. The length adjustment mechanisms are arbitrary; however, in the first embodiment, as an example, the first torso belt 310 has opposite ends coupled to flat rings 33 on the left and right sides of the body part 10, respectively. The second torso belt 320 can be folded back at the flat rings 33 on the left and right sides of the body part 10, respectively. In this case, by adjusting the length of the folded back portions, it becomes possible to adjust the length of a portion of the torso belt 30 surrounding the torso. The folded-back sides and the non-folded-back side of the second torso belt 320 are coupled to each other with hook-and-loop fasteners 55 (only the hook-and-loop fasteners 55 on the non-folded-back side are illustrated). It is noted that, as in the case with the torso belt 30, ends 34 on the folded-back side of the second torso belt 320 may have a thickness and/or a width such that the ends 34 do not escape from the flat rings 33. In this case, at the time of the attachment of the tool 1, even if the torso belt 30 is tensioned in a direction such that the torso belt 30 is loosen, the second torso belt 320 is prevented from escaping from the flat rings 33, which leads to the increased convenience at the attachment. Further, in this case, the user S can adjust the torso belt 30 in a tension increasing direction by pulling the left and right ends 34 in a forward direction with the hands, which eases the adjustment work. Further, pulling the left and right ends 34 in a symmetrical manner is easy, which can reduce the probability that the position of the pocket part 40 is shifted in the left-and-right direction with respect to the center of the user S in the left-and-right direction at the time of the adjustment.

According to the first embodiment, the shoulder belt 20 and the torso belt 30 serve to have the pocket part 40 of the body part 10 come into close contact with the chest of the user S in the attached state of the tool 1. In other words, the user S can have the pocket part 40 of the body part 10 come into close contact with the chest thereof by appropriately adjusting the lengths of the shoulder belt 20 and the torso belt 30 according to the body size thereof. It is noted that the term "close contact" does not always mean direct contact with the naked body of the user S, and thus the close contact may be implemented via the underwear described above. When the pocket part 40 of the body part 10 comes into close contact with the chest of the user S, the biological sensor 70 in the pocket part 40 comes closer to the chest of the user S. Therefore, the measurement accuracy with the biological sensor 70 is increased.

In general, if the user S who has the tool, which carries the biological sensor 70, attached thereto, is in motion during the measurement with the biological sensor 70, it becomes difficult to implement the stable measurement with the biological sensor 70. This is because the position of the biological sensor 70 with respect to the user S easily changes due to the motion of the user S. For example, during a training session, an exercising session, etc., the user S may be in motion (jumping or running, etc., for example) such that acceleration in the up-and-down direction acts on the biological sensor 70, or may be in a posture such that the gravity acts on the biological sensor 70 in a direction away from the user S. It is noted that postures at which the gravity acts on the biological sensor 70 in a direction away from the user S may include the posture at the time of creeping forward, exercising such as push-ups, etc., for example. In such cases, the position of the biological sensor 70 with respect to the user S may easily change.

However, according to the first embodiment, since the tool 1 includes the shoulder belt 20 and the torso belt 30, it becomes possible to keep the pocket part 40 in close contact with the chest of the user S even during a period in which the user S moves relatively strenuously. With this arrangement, it becomes possible to reduce the change in the position of the biological sensor 70 with respect to the user S even during a period in which the user S moves relatively strenuously. As a result of this, according to the first embodiment, stable measurement with the biological sensor 70 can be performed even during a period in which the user S moves relatively strenuously. In the following, such a function of the shoulder belt 20 and the torso belt 30 (i.e., the function of keeping the pocket part 40 in close contact with the chest of the user S, and thus reducing the change in the position of the biological sensor 70 with respect to the user S) is referred to as "a sensor position stabilizing capability".

Here, with reference to Fig. 8 and Fig. 9, preferred configurations to increase the sensor position stabilizing capability are described.

Fig. 8 is a front view illustrating a positional relationship between the torso belt 30 and the pocket part 40 (or a positional relationship between the torso belt 30 and the biological sensor 70 in the pocket part 40).

In Fig. 8, an example of a position C1 corresponding to the center C of the pocket part 40 in the up-and-down direction. Further, in Fig. 8, the biological sensor 70 accommodated in the pocket part 40 is illustrated with dotted lines, and an example of a position G1 in the up-and-down direction corresponding to the center of gravity G of the biological sensor 70. Further, in Fig, 8, an example of coupling areas between the torso belt 30 and the body part 10 are illustrated with ranges W in the up-and-down direction, and a position CB corresponding to the center of the torso belt 30 in the width direction.

It is preferred that the torso belt 30 is coupled to the body part 10 at the position C1 in the up-and-down direction that corresponds to the center C of the pocket part 40 in the up-and-down direction. Specifically, it is preferred that the range W of the coupling areas between the torso belt 30 and the body part 10 is set such that the range W includes the position C1 in the up-and-down direction that corresponds to the center C of the pocket part 40 in the up-and-down direction. Here, as schematically illustrated in Fig. 8, the center C of the pocket part 40 in the up-and-down direction substantially corresponds to the center of gravity G of the biological sensor 70. Thus, if the torso belt 30 is coupled to the body part 10 at the position C1, the probability is high that the body part 10 is supported by the torso belt 30 at the position G1 in the up-and-down direction that corresponds to the center of gravity G of the biological sensor 70. When the body part 10 is supported by the torso belt 30 at the position G1 in the up-and-down direction that corresponds to the center of gravity G of the biological sensor 70, the movement of the body part 10 with respect to the user S can be effectively reduced. This is because the center of the gravity of the body part 10 substantially corresponds to the center of gravity G of the biological sensor 70. Therefore, the sensor position stabilizing capability described above is increased.

Alternatively, from the same viewpoint, the torso belt 30 may be coupled to the body part 10 such that the position CB corresponding to the center in the width direction thereof corresponds to the position C1 in the up-and-down direction corresponding to the center C of the pocket part 40 in the up-and-down direction.

Alternatively, the torso belt 30 may be coupled to the body part 10 at the position G1 in the up-and-down direction that corresponds to the center of gravity G of the biological sensor 70. Specifically, the range W of the coupling areas between the torso belt 30 and the body part 10 may be set such that the range W includes the position G1 in the up-and-down direction that corresponds to the center of gravity G of the biological sensor 70. In this case, the body part 10 can be securely supported by the torso belt 30 at the position G1 in the up-and-down direction that corresponds to the center of gravity G of the biological sensor 70. Therefore, the sensor position stabilizing capability described above is increased. Alternatively, from the same viewpoint, the torso belt 30 may be coupled to the body part 10 such that the position CB corresponding to the center in the width direction thereof corresponds to the position G1 in the up-and-down direction corresponding to the center of gravity G of the biological sensor 70.

Fig. 9 is a schematic cross-sectional view along line A-A in Fig. 8. In Fig. 9, the biological sensor 70 accommodated in the pocket part 40 is schematically illustrated. In Fig. 9, the fastened portions 90 between the front portion 11, the back portion 12 of the body part 10 and the first torso belt 310 are schematically illustrated. It is noted that the fastened portion 90 may be implemented by sewing, for example.

In the first embodiment, in order to increase the sensor position stabilizing capability, as illustrated in Fig. 9, the first torso belt 310 is provided on the body part 10 such that the first torso belt 310 extends across the body part 10 (i.e., the first torso belt 310 passes through the body part 10). For example, the first torso belt 310 is coupled to the back side of the front portion 11 of the body part 10 by sewing, for example, in the area of the pocket part 40 (i.e., the area between the fastened portions 90 on the opposite sides in the left-and-right direction). Further, the first torso belt 310 is formed from an extendable material (a rubber material, for example). The first torso belt 310 in the area of the pocket part 40 can slightly extend in the state in which the biological sensor 70 is accommodated. Thus, because of the force generated by the extended first torso belt 310 that tends to return to the original state, the restraint force against the movement of the biological sensor 70 in the pocket part 40 is increased, which increases the sensor position stabilizing capability.

It is noted that, in the first embodiment, the first torso belt 310 is coupled to the back side of the front portion 11 of the body part 10 in the area of the pocket part 40; however, this is not indispensable. For example, the first torso belt 310 may be coupled to the front side of the front portion 11 of the body part 10 in the area of the pocket part 40, or may be coupled to the front or back side of the back portion 12 of the body part 10 in the area of the pocket part 40.

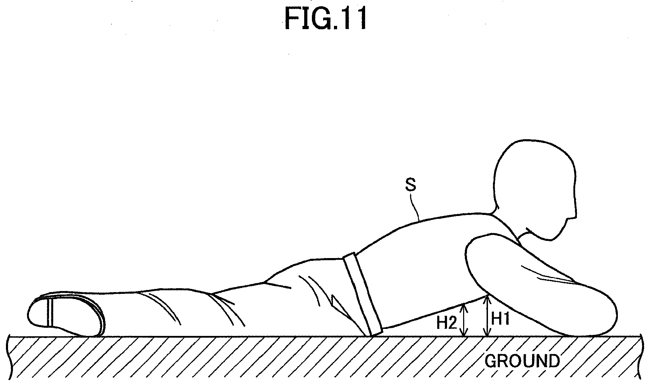

Next, with reference to Fig. 10 and Fig. 11, preferred examples of the biological sensor 70 to be accommodated in the pocket part 40 of the tool 1 according to the first embodiment are described. It is noted that the biological sensor 70 described hereinafter is suited to be used together with the tool 1 as a set (i.e., as a sensing apparatus).

Fig. 10 is a 5-sided view illustrating an example of the biological sensor 70. In Fig. 10, the up-and-down direction is defined in the front view, and the front and back sides are defined in the side view.

The biological sensor 70 includes a casing 710 that defines an outline of the biological sensor 70. The casing 710 is formed by engaging a front side casing part 71 and a back side casing part 72. The casing 710 accommodates items (not illustrated) such as electronic parts, which form sensors, etc., electronic circuits (a substrate), a battery, etc. Here, as an example, the biological sensor 70 has its sensing side on the back side (i.e., the side opposed to the user S) in the accommodated state. In other words, the biological sensor 70 transmits and receives the radio waves via the back side casing part 72.

It is preferred that the casing 710 is thicker (the thickness in a direction corresponding to the front and back direction of the tool 1 in the accommodated state) on the upper side than on the lower side. The casing 710 is in a form of a wedge in the side view such that the thickness h1 on the upper side is greater than the thickness h2 on the lower side. With this arrangement, the biological sensor 70 can be easily inserted into the pocket part 40. Specifically, when the biological sensor 70 is inserted in the pocket part 40, the biological sensor 70 starts to enter the pocket part 40 from the lower part thereof. Thus, as the lower side of the biological sensor 70 becomes thinner, the biological sensor 70 can be inserted in the pocket part 40 more easily. Thus, the thinned lower side of the biological sensor 70 is easier to be inserted in the pocket part 40. Further, since the thickness is different between the upper side and the lower side, the user S can immediately recognize the up-and-down direction of the biological sensor 70 when the user S inserts the biological sensor 70 in the pocket part 40, which eases the insertion of the biological sensor 70 into the pocket part 40. Further, when the user S takes out the biological sensor 70 from the pocket part 40, the user S tends to grasp the upper portion of the biological sensor 70. Thus, the thickened upper side of the biological sensor 70 is easier to be grasped by the user S. Therefore, the thickened upper side of the biological sensor 70 is easier to be taken out from the pocket part 40.

Here, as schematically illustrated in Fig. 11, at the time of creeping forward, the user S is in a posture such that the user S raises the upper half of the body thereof supported with the hands on the ground. Thus, the height from the ground is greater on the side closer to the head of the user S. Further, also at the time of doing push-ups, not limited to the time of creeping forward, the height from the ground similarly becomes greater on the side closer to the head of the user S. Thus, as schematically illustrated in Fig. 11, at the time of creeping forward during the training session, the height H1 of the upper side of the pocket part 40 of the tool 1 from the ground is greater than the height H2 of the lower side of the pocket part 40 from the ground.

Regarding such a scene, according to the biological sensor 70 illustrated in Fig. 10, as described above, since the biological sensor 70 has the lower side thinner than the upper side, it becomes possible to reduce the probability that the lower portion of the biological sensor 70 abuts against the ground even when the user S is in the creeping forward posture, for example, in comparison with the case where the biological sensor 70 has a constant thickness. When the biological sensor 70 abuts against the ground, there may be problems that measurement values include increased noise components, and the user S feels pain. According to the biological sensor 70 illustrated in Fig. 10, such problems can be reduced.

It is preferred that the casing 710 has a non-symmetric form in the up-and-down direction in a front view (i.e., when viewed in a direction corresponding to the front and back direction of the tool 1 in the accommodated state), as illustrated in Fig. 10. Specifically, the upper side surface of the casing 710 is substantially flat, while the lower side surface of the casing 710 is curved in a convex form in the lower direction. With this arrangement, the user S can immediately recognize the up-and-down direction of the biological sensor 70 when the user S inserts the biological sensor 70 in the pocket part 40, which eases the accommodation of the biological sensor 70 in the pocket part 40 with a correct orientation.

It is preferred that the casing 710 is non-symmetric in the up-and-down direction when viewed along the up-and-down direction (in a bottom view or a top view), as illustrated in Fig. 10. For example, the surface of the back side casing part 72 of the casing 710 (i.e., the surface on the back side of the biological sensor 70) is substantially flat, while the surface of the front side casing part 71 (i.e., the surface on the front side of the biological sensor 70) is curved to be convex on the front side. With this arrangement, the user S can immediately recognize the front side and the back side of the biological sensor 70 when the user S inserts the biological sensor 70 in the pocket part 40, which eases the accommodation of the biological sensor 70 in the pocket part 40 with a correct orientation. Further, if the front side casing part 71 and the back side casing part 72 differ in color, the user S can immediately recognize the front side and the back side of the biological sensor 70 when the user S inserts the biological sensor 70 in the pocket part 40, which eases the accommodation of the biological sensor 70 in the pocket part 40 with a correct orientation. Further, if the front side casing part 71 and the back side casing part 72 differ in surface texture, the user S can immediately recognize the front side and the back side of the biological sensor 70 when the user S inserts the biological sensor 70 in the pocket part 40, which eases the accommodation of the biological sensor 70 in the pocket part 40 with a correct orientation. If the surface texture of the front side casing part 71 is smooth while the surface texture of the back side casing part 72 is rough, for example, the user S can recognize the front side and the back side of the biological sensor 70 by touching the biological sensor 70. For this reason, even in a low visibility state, the accommodation of the biological sensor 70 in the pocket part 40 with a correct orientation is easy.

It is preferred that the casing 710 includes, on the upper side surface, an ON/OFF switch 76 (an example of an operation part), an indicator 77 representing a state of the biological sensor 70, and a connector part 78. The ON/OFF switch 76 is operated to turn on/off the biological sensor 70. The indicator 77 includes an LED (Light-Emitting Diode) lamp, for example. The indicator 77 is turned on when the biological sensor 70 is charged, subject to maintenance processes (updating programs, etc., for example), etc. The connector part 78 is provided for the maintenance/setting processes of the biological sensor 70. The connector part 78 is electrically coupled to an external apparatus, if necessary. With this arrangement, the user S can immediately recognize the up-and-down direction of the biological sensor 70 when the user S inserts the biological sensor 70 in the pocket part 40, which eases the accommodation of the biological sensor 70 in the pocket part 40 with a correct orientation.

Here, the pocket part 40 of the tool 1 has the opening on the upper side thereof as described above. Thus, because of the ON/OFF switch 76 being provided on the upper side surface of the casing 710, the user S can easily operate the ON/OFF switch 76 to turn on/off the biological sensor 70 via the opening of the pocket part 40 in the attached state of the tool 1 without taking out the biological sensor 70 from the pocket part 40. Further, because of the indicator 77 being provided on the upper side surface of the casing 710, the user S can easily check the state of the biological sensor 70 via the opening of the pocket part 40 without taking out the biological sensor 70 from the pocket part 40. Further, because of the connector part 78 being provided on the upper side surface of the casing 710, the user S can easily couple the external apparatus to the biological sensor 70 via the opening of the pocket part 40 without taking out the biological sensor 70 from the pocket part 40. It is noted that coupling the external apparatus to the biological sensor 70 may be performed by an assistant other than the user S. However, even in such a case, the coupling operation can be easily performed without taking out the biological sensor 70 from the pocket part 40.

It is noted that, according to the first embodiment, the second torso belt 320 of the torso belt 30 is secured to the back support part 35 such that the second torso belt 320 extends across (passes through) the back support part 35 in the left-and-right direction; however, the second torso belt 320 may be provided such that the second torso belt 320 does not extend across the back support part 35. Specifically, the second torso belt 320 may include a belt part which extends from the left end part of the back support part 35 in the left direction, and another separate belt part which extends from the right end part of the back support part 35 in the right direction. Similarly, according to the first embodiment, the first torso belt 310 is provided on the body part 10 such that the first torso belt 310 extends across (passes through) the body part 10 in the left-and-right direction; however, the first torso belt 310 may be provided such that the first torso belt 310 does not extend across the body part 10.

It is noted that, in the first embodiment described above, it is assumed that the biological sensor 70 to be accommodated in the pocket part 40 is of a single particular type. In this case, the pocket part 40 can be formed based on the outline of the biological sensor 70 of the single particular type, which enables easy insertion and exit of the biological sensor 70 as well as the increased sensor position stabilizing capability. However, the pocket part 40 may be formed such that the pocket part 40 can accommodate a plurality of types of the biological sensor 70 with different outlines, in term of increasing general versatility of the tool 1.

### [Second Embodiment]

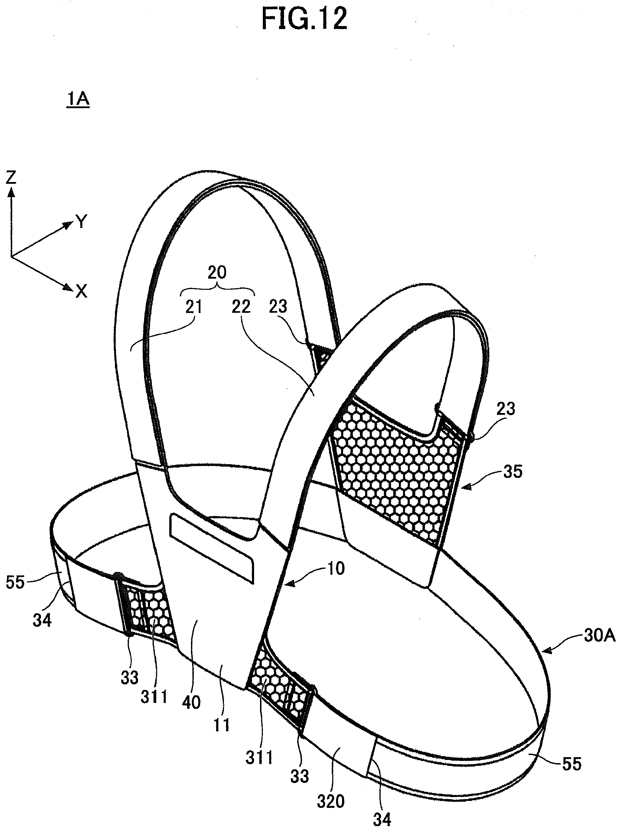

Fig. 12 is a perspective view of a tool 1A according to a second embodiment viewed from a front side.

The tool 1A according to the second embodiment differs from the tool 1 according to the first embodiment described above in that the torso belt 30 is replaced with a torso belt 30A. With respect to the tool 1A according to the second embodiment, elements that may be substantially the same as those according to the first embodiment are given the same reference numerals in Fig. 12, and the explanation thereof is omitted.

The torso belt 30 according to the second embodiment differs from the torso belt 30 according to the first embodiment in a positional relationship with respect to the pocket part 40, that is to say, a coupling position between the torso belt 30A and the body part 10. Specifically, according to the second embodiment, the torso belt 30A is coupled to the body part 10 on the lower side of the body part 10, as illustrated in Fig. 12.

According to the tool 1A according to the second embodiment, substantially the same effects as the tool 1 according to the first embodiment described above are obtained. However, as described above with reference to Fig. 8, the first embodiment is more advantageous than the second embodiment because of the increased probability that the body part 10 is supported by the torso belt 30 at the position G1 in the up-and-down direction corresponding to the center of gravity G of the biological sensor 70.

It is noted that the torso belt 30A according to the second embodiment further differs from the torso belt 30 according to the first embodiment in that the first torso belt 310 is replaced with a first torso belt 311, as illustrated in Fig. 12. The first torso belt 311 may be integrally formed with the back portion 12.

It is noted that even in the second embodiment, various variants in the first embodiment described above are applicable.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a showing of the superiority and inferiority of the invention. Although the embodiment(s) of the present inventions have been described in detail, it should be understood that the various changes, substitutions, and alterations could be made hereto without departing from the spirit and scope of the invention. Further, all or part of the components of the embodiments described above can be combined.

For example, according to the first and second embodiments, the hook-and-loop fasteners (the hook-and-loop fasteners 51, 52, etc.) are used for detachably coupling two members; however, other coupling means such as buttons, magnets, etc., may be used instead of the hook-and-loop fasteners.

Further, according to the first embodiment (the same holds true for the second embodiment), the tool 1 is attached to or detached from the user S in a state (i.e., the state illustrated in Fig. 1, etc.) in which the shoulder belt 20 and the torso belt 30 are not removed from the flat rings 23, 33, respectively. This is advantageous for the attachment of the tool 1 within a short time. However, the shoulder belt 20 and/or the torso belt 30 may include a buckle. It is noted that, if the buckle is provided, the time taken to attach the tool is increased by the time required for the coupling of the buckle.

Further, according to the first embodiment (the same holds true for the second embodiment), the body part 10 is in a form of a trapezoid whose upper base is longer than a lower base as an example of the non-symmetric shape in the up-and-down direction; however, the body part 10 may be in a different form (in a form of a trapezoid whose upper base is shorter than a lower base, for example). However, if the body part 10 is in a form of a trapezoid whose upper base is longer than a lower base, space (a distance in the left-and-right direction) between the coupling portions of the body part 10 with respect to the shoulder belt positions 21 and 22 can be increased. With this arrangement, it becomes possible to increase the stability of the support of the body part 10 with the shoulder belt 20 without substantially increasing the lateral width of the lower portion of the pocket part 40 of the body part 10 with respect to the lateral width of the biological sensor 70.

Further, according to the first embodiment (the same holds true for the second embodiment), only the torso belt 30 is formed from the expendable material; however, at least a part of the shoulder belt 20 may be formed from the expendable material.

Further, according to the first embodiment (the same holds true for the second embodiment), the shoulder belt 20 and the torso belt 30 have adjustable lengths; however, only one of the shoulder belt 20 and the torso belt 30 may have the adjustable length.

It is noted that, with respect to the embodiments described above, the following is disclosed.

### (Notation 1)

A tool configured to be attached to a human body, the tool comprising:
a body part that includes a pocket part capable of accommodating a biological sensor for measurement related to the human body, the body part being configured to be opposed to a chest region of the human body in an attached state of the tool;
a shoulder part coupled to the body part, the shoulder part being configured to rest on a shoulder region of the human body in the attached state of the tool; and
a torso part coupled to the body part, the torso part being configured to extend around a torso region of the human body in the attached state of the tool.

### (Notation 2)

The tool of Notation 1, wherein the shoulder part and the torso part are in a form of a belt.

### (Notation 3)

The tool of Notation 2, wherein at least one of the shoulder part and the torso part has an adjustable length.

### (Notation 4)

The tool of any one of Notations 1 through 3, wherein the torso part is configured to extend around the torso region at a height of the chest region of the human body in the attached state of the tool.

### (Notation 5)

The tool of any one of Notations 1 through 4, wherein the shoulder part and the torso part have different colors.

### (Notation 6)

The tool of any one of Notations 1 through 5, wherein the shoulder part and the torso part have different widths.

### (Notation 7)

The tool of any one of Notations 1 through 6, wherein the body part has different colors on a front surface and a back surface, the back surface being on a side of the human body in the attached state, and the front surface being on an opposite side of the back surface.

### (Notation 8)

The tool of any one of Notations 1 through 7, wherein the pocket part has an opening on a front side of the body part.

### (Notation 9)

The tool of Notation 8, wherein the pocket part includes a lid configured to be opened or closed, the lid being attached to a lower side with respect to the opening in the body part, the lid covering the opening in a closed state of the lid.

### (Notation 10)

The tool of any one of Notations 1 through 9, wherein the body part includes a front portion and a back portion, the back portion having a peripheral area fixed to a back side of the front portion, the back portion having a different mesh fineness with respect to the front portion, and
the pocket part is formed between the front portion and the back portion.

### (Notation 11)

The tool of Notation 10, wherein the back portion is formed with a mesh material.

### (Notation 12)

The tool of any one of Notations 1 through 11, wherein the torso part is coupled to the body part at positions, the positions, in an up-and-down direction, corresponding to a center of the pocket part.

### (Notation 13)

The tool of any one of Notations 1 through 11, wherein the torso part is coupled to the body part at positions, the positions, in an up-and-down direction, corresponding to a center of gravity of the biological sensor in an accommodated state in the pocket part.

### (Notation 14)

The tool of any one of Notations 1 through 13, wherein the torso part includes at least a portion formed from a stretchable material.

### (Notation 15)

The tool of Notation 14, wherein the torso part is provided on the body part such that the torso part extends across the pocket part in a left-and-right direction, and the portion formed from the stretchable material includes a portion across the pocket part.

### (Notation 16)

The tool of any one of Notations 1 through 15, wherein the torso part has a width greater than that of the shoulder part.

### (Notation 17)

The tool of any one of Notations 1 through 16, wherein the body part has an outline such that a width on an upper side is different from that on a lower side, the upper side corresponding to a head side of the human body in the attached state of the tool, the lower side corresponding to a foot side of the human body in the attached state of the tool.

### (Notation 18)

The tool of Notation 17, wherein the body part is in a form of a substantially trapezoid whose upper base is longer than a lower base, the upper base being on the upper side in the attached state of the tool.

### (Notation 19)

The tool of any one of Notations 1 through 18, further comprising a back support part on the opposite side of the body part such that the human body is sandwiched between the back support part and the body part in the attached state.

### (Notation 20)

The tool of Notation 19, wherein the back support part is formed with a mesh material.

### (Notation 21)

The tool of any one of Notations 1 through 20, wherein the biological sensor is configured to emit radio waves and obtain biological information of the human body based on reflected radio waves.

### (Notation 22)

A biological sensor, comprising:
a casing configured to be accommodated in a pocket part of a tool such that the casing can be removed from the pocket, the tool being configured to be attached to a human body, wherein
   the casing is configured such that a thickness of the casing on an upper side is greater than that of the casing on a lower side, the upper side corresponding to a head side of the human body in the attached state of the tool, the lower side corresponding to a foot side of the human body in the attached state of the tool.

### (Notation 23)

The biological sensor of Notation 22, wherein the casing has a non-symmetrical shape with respect to a center in an up-and-down direction, the up-and-down direction being in the accommodated state of the casing.

### (Notation 24)

The biological sensor of Notation 22 or 23, wherein the casing has an upper side surface on which an operation part is provided.

### (Notation 25)

The biological sensor of Notation 22 or 23, wherein the casing has an upper side surface on which a switch, an indicator and a connector part are provided, the switch being operated to turn on/off power, the indicator representing a state of the biological sensor, and the connector part being used for coupling the biological sensor to another apparatus.

## Claims

1. A tool (1, 1A) configured to be attached to a human body, the tool comprising:
a body part (10) that includes a pocket part (40) capable of accommodating a biological sensor (70) for measurement related to the human body, the body part being configured to be opposed to a chest region of the human body in an attached state of the tool;
a shoulder part (20) coupled to the body part, the shoulder part being configured to rest on a shoulder region of the human body in the attached state of the tool; and
a torso part (30, 30A) coupled to the body part, the torso part being configured to extend around a torso region of the human body in the attached state of the tool.

2. The tool of claim 1, wherein the shoulder part and the torso part are in a form of a belt.

3. The tool of claim 2, wherein at least one of the shoulder part and the torso part has an adjustable length.

4. The tool of any one of claims 1 through 3, wherein the torso part is configured to extend around the torso region at a height of the chest region of the human body in the attached state of the tool.

5. The tool of any one of claims 1 through 4, wherein the shoulder part and the torso part have different colors or different widths, or the torso part has a width greater than that of the shoulder part.

6. The tool of any one of claims 1 through 5, wherein the body part has different colors on a front surface and a back surface, the back surface being on a side of the human body in the attached state, the front surface being on an opposite side of the back surface.

7. The tool of any one of claims 1 through 6, wherein the pocket part has an opening (42) on a front side of the body part.

8. The tool of claim 7, wherein the pocket part includes a lid (41) configured to be opened or closed, the lid being attached to a lower side with respect to the opening in the body part, the lid covering the opening in a closed state of the lid.

9. The tool of any one of claims 1 through 8, wherein the body part includes a front portion (11) and a back portion (12), the back portion having a peripheral area fixed to a back side of the front portion, the back portion having a different mesh fineness with respect to the front portion, and
the pocket part is formed between the front portion and the back portion.

10. The tool of claim 9, wherein the back portion is formed with a mesh material, or the torso part includes at least a portion formed from a stretchable material.

11. The tool of any one of claims 1 through 10, wherein the torso part is coupled to the body part at positions, the positions, in an up-and-down direction, corresponding to a center of the pocket part, or
the torso part is coupled to the body part at positions, the positions, in an up-and-down direction, corresponding to a center of gravity of the biological sensor in an accommodated state in the pocket part.

12. The tool of any one of claims 1 through 11, wherein the body part has an outline such that a width on an upper side is different from that on a lower side, the upper side corresponding to a head side of the human body in the attached state of the tool.

13. A biological sensor (70), comprising:
a casing (710) configured to be accommodated in a pocket part (40) of a tool (1, 1A) such that the casing can be removed from the pocket, the tool being configured to be attached to a human body, wherein
the casing is configured such that a thickness of the casing on an upper side is greater than that of the casing on a lower side, the upper side corresponding to a head side of the human body in an accommodated state of the casing and the attached state of the tool.

14. The biological sensor of claim 13, wherein the casing has a non-symmetrical shape with respect to a center in an up-and-down direction, the up-and-down direction being in the accommodated state of the casing.

15. A sensing device, comprising:
a tool (1, 1A) of any one of the claims 1 to 12, and
a biological sensor (70) configured to be accommodated in the tool such that the biological sensor can be removed from the tool.
